(19) Europäisches Patentamt
European Patent Office
Office européen des brevets

(11) Veröffentlichungsnummer: **0 027 978**
**B1**

(12) # EUROPÄISCHE PATENTSCHRIFT

(45) Veröffentlichungstag der Patentschrift: **29.12.82**

(51) Int. Cl.³: **C 07 D 261/08**, A 61 K 31/42

(21) Anmeldenummer: **80106357.9**

(22) Anmeldetag: **18.10.80**

(54) **Neue Aminoderivate des 5-(2-Hydroxystyryl)-isoxazols, Verfahren zu ihrer Herstellung und diese enthaltende therapeutische Mittel.**

(30) Priorität: **26.10.79 DE 2943405**

(43) Veröffentlichungstag der Anmeldung: **06.05.81 Patentblatt 81/18**

(45) Bekanntmachung des Hinweises auf die Patenterteilung: **29.12.82 Patentblatt 82/52**

(84) Benannte Vertragsstaaten: **AT BE CH DE FR GB IT LI LU NL SE**

(56) Entgegenhaltungen:
**AT-B-301 565**
**AT-B-303 055**
**DE-A-2 549 962**
**DE-A-2 818 999**

(73) Patentinhaber: **BASF Aktiengesellschaft, Carl-Bosch-Strasse 38, D-6700 Ludwigshafen (DE)**

(72) Erfinder: **Frickel, Fritz-Frieder, Dr., Silvanerweg 7, D-6705 Deidesheim (DE)**
Erfinder: **Thieme, Peter C., Dr., Dr.-Hans-Hoffmann-Strasse 5, D-6706 Wachenheim (DE)**
Erfinder: **Franke, Albrecht, Dr., Mandelring 11, D-6706 Wachenheim (DE)**
Erfinder: **Theobald, Hans, Dr., Parkstrasse 2, D-6703 Limburgerhof (DE)**
Erfinder: **Lenke, Dieter, Dr., Kekuleplatz 1, D-6700 Ludwigshafen (DE)**
Erfinder: **Gries, Josef, Dr., Roemerweg 43, D-6706 Wachenheim (DE)**

**EP 0 027 978 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Neue Aminoderivate des 5-(2-Hydroxystyryl)-isoxazols, Verfahren zu ihrer Herstellung und diese enthaltende therapeutische Mittel

Die vorliegende Erfindung betrifft neue Aminopropanolderivate des 5-(2-Hydroxystyryl)-isoxazols und ihre Säureadditionssalze, ihre Herstellung und diese Verbindungen enthaltende pharmazeutische Zubereitungen und ihre Verwendung bei der Behandlung der Hypertonie, der coronaren Herzkrankheit und von Herzrhythmusstörungen.

In den AT-PS 301 565 und 303 055 werden verschiedenartig substituierte $\beta$-Aryl-2-aminoalkoxystyrole, die als Arylrest einen carbocyclischen oder heterocyclischen Ring enthalten, beschrieben. Die genannten Verbindungen wirken insbesondere analgetisch, und darüber hinaus sind sie sedierend und muskelrelaxierend wirksam. Wirkungen am Kreislaufsystem, insbesondere $\beta$-sympatholytische und blutdrucksenkende Wirkungen werden nicht beschrieben. Weiterhin sind aus der DE-CS 2 549 962 Isoxazolderivate als Zwischenprodukte zur Herstellung von entsprechenden Phosphor- und Phosphonsäureestern mit insektizider Wirkung bekannt.

Gemäß der DE-OS 2 818 999 werden Aminoderivate von 3-Alkyl-5-(2-hydroxystyryl)-isoxazolen als Arzneimittel mit $\beta$-sympatholytischer und blutdrucksenkender Wirkung vorgeschlagen. Das Stickstoffatom der Aminopropanolseitenkette ist bei diesen Verbindungen durch gesättigte oder ungesättigte Alkylreste oder Cycloalkylreste, die wiederum substituiert sein können, substituiert.

Aufgabe der vorliegenden Erfindung ist es, neue Aminopropanolderivate des 5-(2-Hydroxystyryl)-isoxazols zur Verfügung zu stellen.

Es wurde gefunden, daß Verbindungen der allgemeinen Formel I

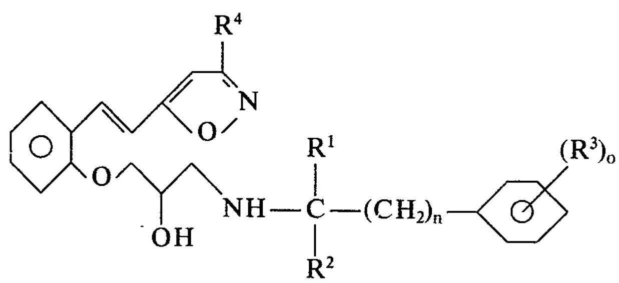

(I)

in der n die Zahlen 1 oder 2, o die Zahlen 1 bis 3, $R^1$ und $R^2$ ein Wasserstoffatom oder eine geradkettige oder verzweigte Alkylgruppe mit 1 bis 5 C-Atomen, $R^3$ ein Wasserstoffatom, eine Hydroxygruppe, ein Halogenatom, eine Alkyl-, Alkoxy- oder Alkylthiogruppe mit 1 bis 5 C-Atomen, gegebenenfalls jeweils in der Alkylgruppe 1- bis 3fach durch Halogenatome oder einfach durch eine Hydroxy- oder Alkoxygruppe mit 1 bis 3 C-Atomen substituiert, eine Alkenyl-, Alkinyl-, Alkinyloxy- oder Cycloalkoxygruppe mit jeweils 2 bis 6 C-Atomen und 3 bis 8 C-Atomen im Ring, eine Aminogruppe, gegebenenfalls ein- oder zweifach mit einer Alkylgruppe mit 1 bis 5 C-Atomen substituiert, bedeuten, wobei entsprechend der Bedeutung von o mehrere Reste $R^3$ gleich oder verschieden sein können, oder $R^3$ bedeutet eine Methylendioxygruppe oder eine Alkylengruppe mit 3 oder 4 Kohlenstoffatomen, und $R^4$ einen Alkylrest mit 1 bis 4 C-Atomen bedeutet und ihre Säureadditionssalze wertvolle pharmakologische Eigenschaften aufweisen. Für $R^3$ sind beispielsweise Halogenatome, Fluor und Chlor, gegebenenfalls substituierte Alkyl-, Alkoxy- oder Alkylthiogruppen, Methyl, Äthyl, Propyl, n-Butyl, tert.-Butyl, Methoxy, Äthoxy, Propoxy, Trifluormethyl, Hydroxymethyl, 1-Hydroxyäthyl, Methoxymethyl, Äthoxymethyl, 1-Methoxyäthyl oder n-Propoxymethyl, ungesättigte Reste beispielsweise Vinyl, Allyl, Propargyloxy, Cycloalkoxyreste, Cyclopentyloxy oder Cyclohexyloxy und Aminogruppen wie die Dimethylaminogruppe.

Wenn $R^3$ eine Trimethylen- oder Tetramethylengruppe bedeutet, handelt es sich um ein Indanyl- oder Tetrahydronaphthylderivat, wie das 4-Indanyl-, 5-Indanyl-, 5,6,7,8-Tetrahydro-1-naphthyl- oder 5,6,7,8-Tetrahydro-2-naphthyl-system.

Von den genannten Bedeutungen sind für $R^1$ und $R^2$ Wasserstoff und Methyl und für $R^3$ Wasserstoff, Chlor, Trifluormethyl, Alkyl mit 1 bis 4 C-Atomen, Methoxy, Hydroxy, Dimethylamino, wobei mehrere Reste $R^3$ gleich oder verschieden sein können, sowie Methylendioxy und für $R^4$ Methyl und Äthyl hervorzuheben.

Demgemäß sind als erfindungsgemäße Verbindungen beispielsweise zu nennen:

3-Methyl-5-[2-[2-hydroxy-3-(2-(3-trifluormethylphenyl)-1,1-dimethyläthylamino)-propoxy]-styryl]-isoxazol

3-Äthyl-5-[2-[2-hydroxy-3-(3-(4-hydroxyphenyl)-1-methylpropylamino)-propoxy]-styryl]-isoxazol

3-Methyl-5-[2-[2-hydroxy-3-(3-(4-hydroxyphenyl)-1-methylpropylamino)-propoxy]-styryl]-isoxazol

3-Methyl-5-[2-[2-hydroxy-3-(2-(3,4-dimethoxyphenyl)-1-äthylamino)-propoxy]-styryl]-isoxazol

3-Methyl-5-[2-[2-hydroxy-3-(3-phenyl-1-methylpropylamino)-propoxy]-styryl]-isoxazol
3-Methyl-5-[2-[2-hydroxy-3-(2-(4-methoxyphenyl)-1-äthylamino)-propoxy]-styryl]-isoxazol
3-Methyl-5-[2-[2-hydroxy-3-(2-(3,4-dimethoxyphenyl)-1-methyläthylamino)-propoxy]-styryl]-isoxazol
3-Methyl-5-[2-[2-hydroxy-3-(2-(4-methoxyphenyl)-1-methyläthylamino)-propoxy]-styryl]-isoxazol
3-Methyl-5-[2-[2-hydroxy-3-(2-(2-methoxyphenyl)-1-methyläthylamino)-propoxy]-styryl]-isoxazol
3-Methyl-5-[2-[2-hydroxy-3-(2-(2-methoxyphenyl)-1,1-dimethyläthylamino)-propoxy]-styryl]-isoxazol
3-Methyl-5-[2-[2-hydroxy-3-(2-(4-methoxyphenyl)-1,1-dimethyläthylamino)-propoxy]-styryl]-isoxazol
3-Methyl-5-[2-[2-hydroxy-3-(3-(4-dimethylaminophenyl)-1-methylpropylamino)-propoxy]-styryl]-isoxazol
3-Methyl-5-[2-[2-hydroxy-3-(3-(4-hydroxyphenyl)-1,1-dimethylpropylamino)-propoxy]-styryl]-isoxazol
3-Methyl-5-[2-[2-hydroxy-3-(2-phenyl-1,1-dimethyläthylamino)-propoxy]-styryl]-isoxazol
3-Methyl-5-[2-[2-hydroxy-3-(3-(3,4-methylendioxyphenyl)-1-methylpropylamino)-propoxy]-styryl]-isoxazol
3-Methyl-5-[2-[2-hydroxy-3-(3-(4-methoxyphenyl)-1,1-dimethylpropylamino)-propoxy]-styryl]-isoxazol
3-Methyl-5-[2-[2-hydroxy-3-(3-(3,4-dimethoxyphenyl)-1-methylpropylamino)-propoxy]-styryl]-isoxazol
3-Methyl-5-[2-[2-hydroxy-3-(2-(3-trifluormethylphenyl)-1-methyläthylamino)-propoxy]-styryl]-isoxazol
3-Methyl-5-[2-[2-hydroxy-3-(2-(4-chlorphenyl)-1,1-dimethyläthylamino)-propoxy]-styryl]-isoxazol
3-Methyl-5-[2-[2-hydroxy-3-(3-(3,4,5-trimethoxyphenyl)-1-methylpropylamino)-propoxy]-styryl]-isoxazol
3-Methyl-5-[2-[2-hydroxy-3-(2-(4-hydroxyphenyl)-1,1-dimethyläthylamino)-propoxy]-styryl]-isoxazol

Weiterhin seien beispielhaft noch erwähnt:

3-Methyl-5-[2-[2-hydroxy-3-(2-(4-hydroxyphenyl)-1-methyläthylamino)-propoxy]-styryl]-isoxazol
3-Methyl-5-[2-[2-hydroxy-3-(2-(2-hydroxyphenyl)-1,1-dimethyläthylamino)-propoxy]-styryl]-isoxazol

Die erfindungsgemäßen Verbindungen können hergestellt werden, indem man ein 3-Alkyl-5-styryl-isoxazol der allgemeinen Formel (II)

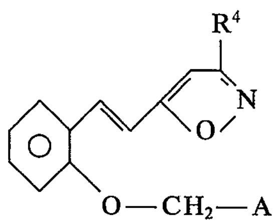

(II)

in der $R^4$ die für Formel I angegebenen Bedeutungen hat und A den Rest

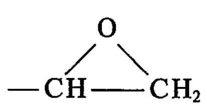 oder 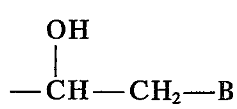

wobei B für eine nukleofuge Abgangsgruppe steht, bedeutet, mit einem Amin der allgemeinen Formel (III)

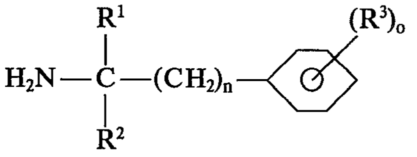

(III)

in der $R^1$, $R^2$, $R^3$, n und o die für Formel I angegebenen Bedeutungen haben, zweckmäßigerweise in einem Lösungsmittel und gegebenenfalls in Gegenwart eines säurebindenden Mittels in an sich bekannter Weise umsetzt und die erhaltene Verbindung gegebenenfalls in das Säureadditionssalz einer physiologisch verträglichen Säure überführt.

Die Abgangsgruppe B stellt bevorzugt ein Halogenatom, insbesondere Chlor, Brom oder Jod, dar. Weiterhin kommen beispielsweise als nukleofuge Abgangsgruppen aromatische oder aliphatische Sulfonsäurereste in Betracht, wie der p-Toluolsulfonsäure-, der p-Brombenzolsulfonsäure- oder der Methansulfonsäurerest. Die Umsetzungen werden bei Temperaturen von 10 bis 120°C, d. h. bei Raumtemperaturen oder bei höheren Temperaturen, zweckmäßig bei Temperaturen von 50 bis 120°C, durchgeführt. Die Umsetzungen können unter Atmosphärendruck oder in einem geschlossenen Gefäß unter erhöhtem Druck gegebenenfalls unter Erwärmung auf den angegebenen Temperaturbereich durchgeführt werden.

Die Ausgangsverbindungen können direkt, d. h. ohne Zugabe eines Verdünnungs- oder Lösungsmittels, umgesetzt werden. Zweckmäßigerweise werden die Umsetzungen jedoch in Gegenwart eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, von Benzol oder eines Alkylbenzols, wie Toluol oder Xylol, oder eines aliphatischen Kohlenwasserstoffs, wie Hexan, Heptan oder Octan, eines niederen aliphatischen Ketons, wie Aceton, Methyläthylketon oder Methylisobutylketon, eines Dialkylformamids, wie Dimethyl- oder Diäthylformamid, von Dimethylsulfoxid oder in Gegenwart von Wasser oder in Mischungen der genannten Lösungsmittel, durchgeführt.

Bevorzugte Lösungsmittel bei der Umsetzung von 3-Methyl-5-[2-(2,3-epoxy-propoxy)-styryl]-isoxazol mit einem der oben angeführten Amine (III) sind niedere Alkohole, insbesondere Äthanol oder Isopropanol, wobei die Umsetzung bevorzugt bei Temperaturen von 50 bis 120°C und bei Normaldruck durchgeführt wird. Bei der nukleophilen Substitution eines Restes B sind als Lösungsmittel ein niederes aliphatisches Keton, wie Aceton oder Methyl-isobutylketon, ein cyclischer Äther, insbesondere Tetrahydrofuran oder Dioxan, oder ein Dialkylformamid, wie Dimethylformamid, und Temperaturen von 90 bis 120°C bevorzugt. Gegebenenfalls wird die Reaktion in Gegenwart einer katalytischen Menge Natrium- oder Kaliumjodid durchgeführt.

Es sei erwähnt, daß als Ausgangsverbindung der Formel II gegebenenfalls auch eine Mischung des Epoxids mit einem Halogenhydrin in Betracht kommt, da bei der technischen Herstellung der Ausgangsverbindungen der Formel II derartige Gemische unter Umständen entstehen können.

Bei einer zweckmäßigen Ausführungsform zur nukleophilen Substitution des Restes B durch das verwendete Amin wird die Umsetzung in Gegenwart einer Base als säurebindendes Mittel durchgeführt. Bevorzugte Basen sind Alkalimetallhydroxide, -carbonate, -hydrogencarbonate, -alkoholate oder ein tertiäres organisches Amin, wie Pyridin, oder ein Trialkylamin, wie Trimethylamin oder Triäthylamin. Von den Alkaliverbindungen kommen insbesondere die des Natriums und Kaliums in Betracht. Dabei wird die Base in stöchiometrischer Menge oder in geringem Überschuß verwendet. Gegebenenfalls ist es zweckmäßig, das zur Umsetzung verwendete Amin III in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z. B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel. Überführen in eine Säureadditionsverbindung oder durch Säulenchromatographie.

Die Ausgangsverbindungen der Formel (II) können durch Alkylierung eines 3-Alkyl-5-(2-hydroxy-styryl)-isoxazols, die beispielsweise in einer Wittig-Reaktion aus einem Dialkyl-isoxazolyl-5-methylenphosphonat und einem an der OH-Gruppe mit einer Schutzgruppe versehenen Salicylaldehyd gemäß Patentanmeldung P 28 18 998.2, hergestellt werden, mit einem Epihalogenhydrin oder einem $\alpha,\omega$-Dihalogen-2-propanol erhalten werden. Als Epihalogenhydrine kommen Epichlorhydrin, Epibromhydrin und Epijodhydrin und als $\alpha,\omega$-Dihalogen-2-propanol kommen insbesondere 1,3-Dichlor-2-propanol und 1,3-Dibrom-2-propanol in Betracht.

Die Umsetzung der 3-Alkyl-5-(2-hydroxy-styryl)-isoxazole zur Herstellung der Ausgangsverbindungen der Formel III werden zweckmäßigerweise bei Temperaturen von 0 bis 120°C und unter Normaldruck oder in einem geschlossenen Gefäß unter erhöhten Drucken durchgeführt. Die Umsetzungen werden zweckmäßigerweise in einem inerten Verdünnungs- oder Lösungsmittel, z. B. einem niederen aliphatischen Keton, wie Aceton, Methyläthylketon oder Methyl-isobutylketon, einem niederen Alkohol mit 1 bis 4 C-Atomen, wie Methanol, Äthanol, Propanol oder Butanol, einem niederen aliphatischen oder cyclischen Äther, wie Diäthyläther, Tetrahydrofuran oder Dioxan, einem Dialkylformamid, wie Dimethylformamid oder Diäthylformamid, oder Dimethylsulfoxid und Hexamethylphosphortriamid oder mit überschüssigem Alkylierungsmittel als Verdünnungs- oder Lösungsmittel durchgeführt.

Bevorzugt werden die Umsetzungen in Gegenwart einer Base als säurebindendes Mittel

vorgenommen. Geeignete Basen sind Alkalimetallcarbonate, -hydrogencarbonate, -hydroxide, -hydride oder -alkoholate, insbesondere des Natriums und Kaliums, basische Oxide, wie Aluminiumoxid oder Calciumoxid, organische tertiäre Basen, wie Pyridin, Piperidin oder niedere Trialkylamine, wie Trimethyl- oder Triäthylamin. Dabei können die Basen im Verhältnis zum eingesetzten Alkylierungsmittel in katalytischer Menge oder in stöchiometrischer Menge bzw. in geringem Überschuß verwendet werden.

Bevorzugt werden die 3-Alkyl-5-(2-hydroxy-styryl)-isoxazole mit Epibromhydrin oder 1,2-Dibrompropanol-2 in einem polaren aprotischen Lösungsmittel, insbesondere Dimethylsulfoxid, in Gegenwart von mindestens einem Moläquivalent Base, insbesondere Natriumhydrid, bezogen auf das Alkylierungsmittel, bei Temperaturen von 0 bis 50° C umgesetzt.

Analog des in Liebigs Annalen der Chemie 1976, Seiten 221 bis 224, beschriebenen Verfahrens für die Umsetzung von Phenol mit 1,3-Dichlor-2-propanol können auch 3-Alkyl-5-(2-hydroxy-styryl)-isoxazole mit der äquivalenten Menge 1,3-Dichlor-2-propanol in wäßriger Natronlauge bei Temperaturen um 50° C umgesetzt werden.

Die Ausgangsverbindungen der allgemeinen Formel II, in denen A den Rest

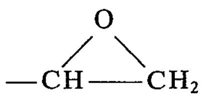

bedeutet, können auch erhalten werden, indem man einen Methanphosphonsäureester der allgemeinen Formel IV

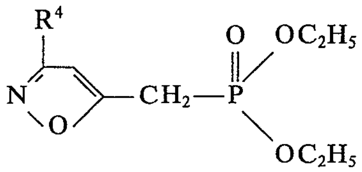

(IV)

mit dem in J. Chem. Soc. London 1974, 1571 bis 1577 beschriebenen o-(2,3-Epoxypropoxy)-benzaldehyd umsetzt.

Zur Reaktion von o-(2,3-Epoxypropoxy)-benzaldehyd mit einem Phosphoran der Formel IV kommen als Reaktionsmedium inerte organische Lösungsmittel in Betracht, wie z. B. ein niederer gesättigter Dialkyläther, Dialkylglykoläther oder cyclischer Äther, wie Diäthyläther, 1,2-Dimethyloxyäthan, Tetrahydrofuran oder Dioxan, Benzol oder ein Alkylbenzol, wie Toluol oder Xylol, ein aliphatischer Kohlenwasserstoff, wie Hexan, Heptan oder Octan, Dimethylsulfoxid, Dimethylformamid oder Mischungen der genannten Lösungsmittel. Die Umsetzungen werden zwischen 0°C und den Siedetemperaturen der verwendeten Lösungsmitteln, zweckmäßigerweise bei Raumtemperatur, innerhalb von 1 bis 48 Stunden, vorzugsweise 1 bis 16 Stunden und vorteilhaft in einer Stickstoffatmosphäre durchgeführt. Geeignete Basen für diese Wittig-Horner-Reaktion sind Alkalimetallhydride, -amide oder -alkoholate, insbesondere die des Natriums und Kaliums, vorzugsweise verwendet man Natriummethylat.

Gemäß einem weiteren Herstellungsverfahren können die erfindungsgemäßen Verbindungen erhalten werden, indem man einen Methanphosphonsäureester der Formel IV mit einer Verbindung der Formel V in Gegenwart einer Base und zweckmäßigerweise in Gegenwart eines Lösungsmittels in an sich üblicher Weise unter den Bedingungen der Wittig-Horner-Reaktion zur Reaktion bringt.

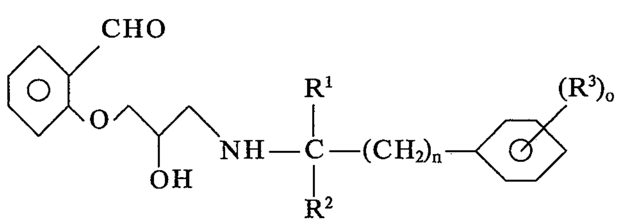

(V)

Diese Umsetzungen können beispielsweise gemäß den in der DE-OS 1 939 809 beschriebenen Bedingungen erfolgen.

Die Wittig-Horner-Reaktionen werden zweckmäßig in einem inerten Verdünnungs- oder Lösungsmittel, z. B. einem niederen gesättigten Dialkyläther, Dialkylglykoläther oder cyclischen Äther, wie Diäthyläther, 1,2-Dimethoxyäthan, Tetrahydrofuran oder Dioxan, Benzol oder einem Alkylbenzol, wie Toluol oder Xylol, einem aliphatischen Kohlenwasserstoff, wie Hexan, Heptan oder Octan, Dimethylsulfoxid oder in Mischungen der genannten Lösungsmittel durchgeführt. Die Umsetzungen werden zweckmäßigerweise bei Umgebungstemperatur oder durch Erwärmen auf 30 bis 80°C ausgeführt. Geeignete Basen sind Alkalimetallhydride, -amide oder -alkoholate, insbesondere die des

Natriums und Kaliums und Butyl- und Phenyllithium.

Die erfindungsgemäßen Verbindungen können aufgrund der Kohlenstoff-Kohlenstoff-Doppelbindung als cis-trans-Isomerengemisch vorliegen. Mit üblichen physikalisch-chemischen Methoden, wie fraktionierender Kristallisation, Chromatographie oder Sublimation, können die Isomeren aufgetrennt werden.

Die erfindungsgemäßen Verbindungen der Formel (I) besitzen am Kohlenstoffatom zwei der aliphatischen Aminopropanolseitenkette ein Chiralitätszentrum und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsäure, Ditoluolweinsäure oder 3-Brom-campher-8-sulfonsäure, in die optisch aktiven Antipoden getrennt werden können.

In einigen Fällen weisen die erfindungsgemäßen Verbindungen der Formel (I) je nach Auswahl der verwendeten Amine (III) ein zweites asymmetrisches Kohlenstoffatom auf und können dann als Diastereomerengemische vorliegen, das in bekannter Weise mit physikalisch-chemischen Methoden in Diastereomerenpaare getrennt werden kann. Optisch reine Formen der erfindungsgemäßen Verbindungen mit zwei Chiralitätszentren sind bei Verwendung von optisch aktiven Aminen der allgemeinen Formel (II) und anschließender Trennung der beiden Diastereomeren, beispielsweise durch fraktionierende Kristallisation oder Chromatographie erhältlich.

Gegebenenfalls werden die erhaltenen erfindungsgemäßen erfindungsgemäßen Verbindungen in das Säureadditionssalz einer physiologisch verträglichen Säure überführt. Als übliche physiologisch verträgliche organische oder anorganische Säuren kommen beispielsweise in Betracht: Salzsäure, Bromwasserstoffsäure, Phosphorsäure oder Schwefelsäure, und als organische Säuren beispielsweise Maleinsäure, Fumarsäure, Milchsäure, Weinsäure, Apfelsäure, Zitronensäure, Salicylsäure, Adipinsäure oder Benzoesäure oder können aus Fortschritte der Arzneimittelforschung, Band 10, Seiten 224 bis 225, Birkhäuser Verlag, Basel und Stuttgart, 1966, oder aus Journal of Pharmaceutical Science, Volume 66, 1—5 (1977), entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Äthanol oder Propanol, oder einem niederen Keton, wie Aceton, Methyläthylketon oder Methylisobutylketon oder einem Äther, wie Diäthyläther, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Aminopropanol-Derivate der allgemeinen Formel (I) durch Auflösen der freien Basen der allgemeinen Formel (I) in einer wäßrigen Säurelösung hergestellt werden.

Die erfindungsgemäßen Verbindungen und ihre physiologisch verträglichen Säureadditionssalze weisen wertvolle pharmakologische Eigenschaften auf.

Sie sind pharmakodynamisch als β-Sympatholytika mit akut blutdrucksenkender Wirkung zu charakterisieren. Dieser Wirkungstyp ist von den bekannten β-Sympatholytika, z. B. Propanolol, zu unterscheiden. Propanolol wirkt im akuten Versuch nicht hypotensiv. Aufgrund der genannten Effekte sind die Verbindungen pharmakotherapeutisch besonders geeignet zur Behandlung der Hypertonie, der coronaren Herzkrankheit (Angina Pectoris) und von Herzrhythmusstörungen.

Die Untersuchungen der pharmakodynamischen Eigenschaften werden nach folgenden Methoden vorgenommen:

### 1. β-sympatholytische Wirkung

Isoproterenol (0,1 µg/kg i. v.) verursacht an despinalisierten Ratten (Stamm: Sprague-Dawley ♂, Gewicht: 200 bis 240 g) Herzfrequenzsteigerungen um durchschnittlich $121 \pm 2{,}1\ min^{-1}$ bei einer Ausgangsfrequenz von etwa $268 \pm 3{,}9\ min^{-1}$ (N = 100).

β-Sympatholytika hemmen diese Frequenzsteigerungen spezifisch und dosisabhängig. Die Applikation der getesteten Substanzen erfolgt 5 min vor Isoproterenol. Als ED 50% wird die Dosis ermittelt, welche die Isoproterenol-Tachycardie um 50% hemmt.

### 2. Blutdrucksenkende Wirkung an der narkotisierten Ratte

Zur Testung der blutdrucksenkenden Wirkung erhalten männliche Sprague-Dawley-Ratten (Gewicht: 230—280 g) in Urethan-Narkose (1,78 g/kg i. p.) die Substanzen intravenös appliziert.

Die Messung des Blutdrucks in der A. carotis erfolgt über Statham-Tranducer. Als ED 20% wird die Dosis ermittelt, welche den mittleren Carotisdruck um 20% senkt.

3. Akute Toxizität an der Maus

Zur Bestimmung der akuten Toxizität (LD 50) werden die Substanzen weiblichen NMRI-Mäusen (Gewicht: 22 bis 26 g) intraperitoneal appliziert.

Die Berechnung der wirksamen Dosen (s. 1. und 2.) erfolgte aus der linearen Beziehung zwischen den Logarithmen der Dosen und der Wirkung mit Hilfe der Regressionsanalyse. Die LD 50 (3.) wurden mit Hilfe der Probit-Analyse bestimmt. Als Referenzsubstanz diente das bekannte $\beta$-Sympatholytikum Propanolol.

Die $\beta$-sympatholytische Wirksamkeit (s. Tabelle) der Beispiele 3 und 4 entspricht etwa derjenigen vom Propanolol. Neben der $\beta$-sympatholytischen Wirkung besitzen die erfindungsgemäßen Verbindungen im Gegensatz zu Propanolol eine akut blutdrucksenkende Wirkung. Die Wirkung ist dosisabhängig. Nach i. v. Injektion von 0,49 mg/kg, Beispiel 3, oder 0,7 mg/kg, Beispiel 4, wird an der Ratte eine 20%ige Blutdrucksenkung ausgelöst. Dagegen senkt Propanolol den Blutdruck bis zu Dosen von 2,15 mg/kg nicht. Erst die subletale Dosis 4,64 mg/kg senkt ihn um durchschnittlich 36%.

Außer den pharmakotherapeutisch wichtigen Wirkungen Blutdrucksenkung und $\beta$-Sympatholyse zeigen die genannten Substanzen eine geringe Toxizität. Die letale Dosis ist bei Beispiel 3 24 600mal, bei Beispiel 4 29 900mal höher als die $\beta$-sympatholytisch wirksame. Bei Propanolol ist die letale Dosis nur 8500mal höher als die wirksame.

| Beispiel Nummer | Betasympatholyt. Wirkung[1] | | Blutdruck-senkende W.[2] | Letalität[4] | Q[5] |
|---|---|---|---|---|---|
| | ED 50% | R. W. | ED 20% | LD 50 | |
| 3 | 0,0106 | 1,20 | 0,49 | 261 | 24 600 |
| 4 | 0,0215 | 0,59 | 0,70 | 642 | 29 900 |
| Propanolol | 0,0127 | 1,00 | [3] | 108 | 8 500 |

[1] Ratte despinalisiert. Appl.: i. v.
ED 50%= Dosis [mg/kg], welche die Steigerung der Herzfrequenz durch Isoproterenol um 50% hemmt.
R. W. = Relative Wirksamtkeit. Propanolol = 1,00.

[2] Ratte. Urethan-Narkose. Appl.: i. v.
ED 20%= Dosis [mg/kg], welche den Blutdruck um 20% senkt.

[3] Bei 2,15 mg/kg Blutdrucksteigerung (11%); 4,64 mg/kg Blutdrucksenkung (36%); 10 mg/kg 2/6 Tiere gestorben.

[4] Maus. Appl.: i. p. LD 50 mg/kg.

[5] $Q = \frac{LD\ 50}{ED\ 50\%}$

Gegenstand der vorliegenden Erfindung sind demnach auch therapeutische Mittel oder Zubereitungen, die neben üblichen Träger- und Verdünnungsmitteln eine Verbindung der Formel (I) als Wirkstoff enthalten, sowie die Verwendung der neuen Verbindungen zu therapeutischen Zwecken.

Die therapeutischen Mittel bzw. Zubereitungen werden mit den üblichen flüssigen oder festen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Calciumcarbonat, Calciumphosphat oder Milchzucker, Sprengmittel wie Mais, Stärke oder Alginsäure, Bindemitteln wie Stärke oder Gelatine, Gleitmittel wie Magnesiumstearat oder Talk und/oder Mitteln zur Erzielung des Depoteffektes wie Carboxypolymethylen, Carboxymethylcellulose, Celluloseacetatphthalat oder Polyvinylacetat erhalten werden. Die Tabletten

können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können. Lösungen oder Suspensionen mit den erfindungsgemäßen Wirkstoffen können zusätzlich geschmacksverbessernde Mittel wie Saccharin, Cyclamat oder Zucker sowie z. B. Aromastoffe wie Vanillin oder Orangenextrakt, enthalten. Sie können außerdem Suspendierhilfsstoffe, wie Natriumcarboxymethylcellulose oder Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln, wie Neutralfetten oder Polyäthylenglykol bzw. deren Derivaten, herstellen.

Als Einzeldosis der erfindungsgemäßen Verbindungen kommen am Menschen 1 bis 100 mg, vorzugsweise 3 bis 50 mg, in Betracht.

Die vorliegende Erfindung wird durch die nachfolgenden Ausführungsbeispiele näher erläutert.

## Herstellung von Vorprodukten

### Verbindung I

### o-(α-Methoxy-äthoxy)-benzaldehyd

a) 610 g (5 Mol) Salicylaldehyd werden in 1,5 l Xylol gelöst; zu dieser Lösung werden bei 40 bis 50° C 900 g (5 Mol) einer 30%igen $NaOCH_3$-Lösung in Methanol zugetropft. Anschließend wird aufgeheizt und das Methanol abdestilliert, welches im Reaktionskolben nach und nach durch die gleiche Menge Xylol ersetzt wird. Es wird so lange erhitzt, bis das Xylol abzudestillieren beginnt (ca. 130° C am Destillationsübergang). Dann wird die Suspension des Na-Salzes des Salicylaldehyds auf 60° C abgekühlt und wie unter c) beschrieben weiter umgesetzt.

b) 200 ml Xylol werden mit einer Spatelspitze Hydrochinon versetzt und auf −20 bis −30° C abgekühlt und 290 g (5 Mol) Vinyl-methyläther einkondensiert. Anschließend werden bei −30° C 183 g (5 Mol) HCl eingegast und die Lösung durch Stehenlassen auf Raumtemperatur erwärmt. Die Lösung des erhaltenen 1-Chloräthyl-methyläthers wird wie unter c) beschrieben weiter umgesetzt.

c) Die wie unter b) beschrieben hergestellte Lösung des 1-Chloräthylmethyläthers wird zu der 60° C heißen Lösung des Na-Salzes des Salicylaldehyds (siehe a) zugetropft und noch ca. 1 1/2 Stunden bei 60° C weitergerührt, gegebenenfalls wird mit Hilfe von 30%iger $NaOCH_3$-Lösung ein pH-Wert von 8 bis 9 eingestellt und über Nacht bei Raumtemperatur weitergerührt.
Anschließend wird vom ausgefallenen Natriumchlorid abfiltriert, nachgewaschen mit Xylol und am Rotationsverdampfer das Xylol abdestilliert. Der verbleibende Rückstand wird bei 2 mm Hg über eine Kolonne destilliert. Es werden 690 g o-(α-Methoxy-äthoxy)-benzaldehyd vom $Kp_2$: 94 bis 96° C gewonnen.

### Verbindung II

### (3-Methylisoxazolyl-5)-methanphosphonsäurediäthylester

445 g 5-Chlormethyl-3-methylisoxazol und 674 g Phosphorigsäuretriäthylester werden langsam auf 150° C erhitzt und 4 Stunden bei dieser Temperatur belassen. Nach der Destillation erhält man 546 g (69% der Theorie) (3-Methylisoxazolyl-5)-methanphosphonsäurediäthylester vom Sdp. 118 bis 121° C/0,3 Torr.

$^1$H-NMR-Spektrum ($CHCl_3$, TMS intern):
$\tau$ = 3,85 (d, J = 3 Hz, 1H), 4,17 (m, J = 8 Hz, 4H), 6,67 (d, J = 22 Hz, 2H), 7,72 (s, 3H), 8,67 (t, J = 8 Hz, 6H).

$C_9H_{16}NO_4P$ (233,21)

| | | | | |
|---|---|---|---|---|
| Ber.: | C 46,35% | H 6,91% | N 6,01% | P 13,28% |
| Gef.: | C 45,9% | H 7,0% | N 6,0% | P 13,0% |

Verbindung III

(3-Äthylisoxazolyl-5)-methanphosphonsäurediäthylester

15 g 5-Chlormethyl-3-äthyl-isoxazol und 18 g Triäthylphosphit werden langsam auf 150° C aufgeheizt und 2[1]/2 Stunden bei dieser Temperatur belassen. Nach dem Abkühlen wird der Rückstand unter vermindertem Druck destilliert. Man erhält 18,2 g (3-Äthylisoxazolyl-5)-methanphosphonsäurediäthylester vom Sdp. 120 bis 121° C/0,2 mm Hg (Ausbeute: 71,2%).

$^1$H-NMR-Spektrum ($CDCl_3$, TMS intern):
$\tau$ = 3,85 (d, J = 3 Hz, 1H), 4,17 (m, J = Hz, 4H), 6,60 (d, H = 20 Hz, 2H), 7,35 (q, J = Hz, 2H), 8,50—8,93 (m, 9H).

$C_{10}H_{18}NO_4P$ (247,23)

| | | | | |
|---|---|---|---|---|
| Ber.: | C 48,58% | H 7,34% | N 5,67% | P 12,53% |
| Gef.: | C 48,4% | H 7,1% | N 5,7% | P 12,3% |

Die weiteren Phosphonester werden analog hergestellt.

(3-Isopropyl-sioxazolyl-5)-methanphosphonsäurediäthylester
$Kp_{0,3}$: 7—122° C, Ausbeute 73%.
(3-tert.-Butyl-isoxazolyl-5)-methanphosphonsäurediäthylester
$Kp_{0,3}$: 126—132° C, Ausbeute 88%.

Herstellung von Ausgangsverbindungen

Beispiel I

3-Methyl-5-(2-hydroxy-styryl)-isoxazol

8,8 g (0,2 Mol) 55% Natriumhydridsuspension in Paraffinöl werden in 100 ml absolutem Dimethylsulfoxid vorgelegt. Dazu werden 47 g (0,2 Mol) Diäthyl-(3-methyl-isoxazolyl-5)-methylenphosphat bei Raumtemperatur getropft. Man läßt 30 min nachrühren und tropft anschließend unter Rühren 36 g (0,2 mol) o-(1-Methoxyäthoxy)-benzaldehyd zu und läßt das Reaktionsgemisch für 24 Std. bei Raumtemperatur rühren. Man gießt auf 1 l Eiswasser und extrahiert 3mal mit je 80 ml Methylenchlorid. Die vereinigten organischen Phasen werden mit Natriumsulfat getrocknet und am Rotationsverdampfer eingeengt. Der ölige Rückstand wird in 80 ml Methanol und 10 ml Wasser gelöst und mit 2 ml 5 n HCl versetzt und für 10 min nachgerührt. Dann wird langsam ein Überschuß an Wasser zum Gemisch gegeben, bis ein Niederschlag ausfällt. Dieser wird abgesaugt, mit Wasser gewaschen und aus Äthanol umkristallisiert. 19 g (47% d. Th.) farblose Kristalle. Fp. 236—238° C.

$C_{12}H_{11}NO_2$ (201)

| | | | |
|---|---|---|---|
| Ber.: | C 71,6 | H 5,5 | N 7,0 |
| Gef.: | C 71,8 | H 5,5 | N 6,8 |

Beispiel II

3-Äthyl-5-(2-hydroxy-styryl)-isoxazol

Gemäß I werden 6 g (0,02 Mol) Diäthyl-(3-äthyl-isoxazol-5)-methylenphosphat und 4,4 g (0,02 Mol) o-(1-Methoxyäthoxy)-benzaldehyd umgesetzt und aus Isopropanol umkristallisiert 1,7 g (32% d. Th.) farblose Kristalle. Fp. 175—176° C.

$C_{13}H_{13}NO_2$ (215)

| | | | |
|---|---|---|---|
| Ber.: | C 72,5 | H 6,1 | N 6,5 |
| Gef.: | C 72,5 | H 6,2 | N 6,6 |

## Beispiel III

### 3-Isopropyl-5-(2-hydroxy-styryl)-isoxazol

Gemäß I werden 32 g (0,12 Mol) Diäthyl-(3-isopropyl-isoxazolyl-5)-methylen-phosphonat und 22 g (0,12 Mol) o-(1-Methoxy-äthoxy)-benzaldehyd umgesetzt und aus Toluol umkristallisiert. 20 g (73% d. Th.) farblose Kristalle. Fp. 129—133° C.

$C_{16}H_{15}NO_2$ (229)

| | | | |
|---|---|---|---|
| Ber.: | C 73,3 | H 6,6 | N 6,1 |
| Gef.: | C 73,7 | H 6,7 | N 5,8 |

## Beispiel IV

### 3-tert.-Butyl-5-(2-hydroxy-styryl)-isoxazol

Gemäß I werden 35 g (0,13 Mol) Diäthyl-(3-tert.-Butyl-isoxazolyl-5)-methylen-phosphonat und 23 g (0,13 Mol) o-(1-Methoxy-äthoxy)-benzaldehyd umgesetzt und am Toluol umkristallisiert. 24,8 g (78% d. Th.) farblose Kristalle. Fp. 152—155° C.

$C_{15}H_{17}NO_2$ (243)

| | | | |
|---|---|---|---|
| Ber.: | C 74,0 | H 7,0 | N 5,8 |
| Gef.: | C 73,4 | H 7,3 | N 5,5 |

## Beispiel V

### 3-Methyl-5-[2-(2,3-epoxypropoxy)-styryl]-isoxazol

6,44 g 55%iges Natriumhydrid in Paraffinöl (0,15 Mol) werden in 200 ml absolut Dimethylsulfoxid vorgelegt und tropfenweise bei Raumtemperatur mit 30 g (0,15 Mol) 3-Methyl-5-(2-hydroxy-styryl)-isoxazol, in 50 ml Dimethylsulfoxid gelöst, versetzt. Nach beendeter Wasserstoffentwicklung werden 20,2 g (0,15 Mol) Epibromhydrin zugetropft und das Reaktionsgemisch für 20 Stunden bei Raumtemperatur gerührt. Man gibt das Gemisch auf 1,5 l Eiswasser, der feste Rückstand wird abgesaugt und aus Isopropanol umkristallisiert. 26,2 g (68% d. Th.) farblose Kristalle. Fp. 99—100° C.

$C_{15}H_{15}NO_3$ (257)

| | | | |
|---|---|---|---|
| Ber.: | C 70,0 | H 5,9 | N 5,4 |
| Gef.: | C 70,0 | H 5,9 | N 5,5 |

## Beispiel VI

### 3-Äthyl-5-[2-(2,3-epoxypropoxy)- tyryl]-isoxazol

Wird analog V aus 5,1 g 55%igem Natriumhydrid (0,116 Mol), 25,0 g (0,116 Mol) 3-Äthyl-5-(2-hydroxystyryl)-isoxazol und 15,9 g (0,116 Mol) Epibromhydrin hergestellt. Das Reaktionsgemisch wird auf Kochsalzlösung gegossen und mit Diäthyläther ausgeschüttelt. Die ätherische Lösung wird mit wasserfreiem Natriumsulfat getrocknet und eingeengt. Man erhält 29,2 g (93% d. Th.) farbloses Öl.

## Beispiel VII

### 3-Isopropyl-5-[2-(2,3-epoxypropoxy)-styryl]-isoxazol

Wird analog VI aus 3,4 g 55%igem Natriumhydrid (0,078 Mol), 18 g (0,078 Mol) 3-Isopropyl-5-(2-hydroxystyryl)-isoxazol und 10,8 g (0,078 Mol) Epibromhydrin hergestellt. 21,5 g (97% d. Th.) farbloses Öl.

## Beispiel VIII

### 3-tert.-Butyl-5-[2-(2,3-epoxypropoxy)-styryl]-isoxazol

Wird analog VI aus 3,8 g 55%igem Natriumhydrid (0,086 Mol), 21 g (0,086 Mol) 3-tert.-Butyl-5-(2-hydroxystyryl)-isoxazol und 11,8 g (0,086 Mol) Epibromhydrin hergestellt. 25,0 g (97% d. Th.) farbloses Öl.

## Beispiel IX

1,0 g 3-Methyl-5-[2-(2,3-epoxypropoxy)-styryl]-isoxazol wird in 20 ml 3-N-ätherischer Chlorwasserstofflösung gelöst und 12 Stunden bei Raumtemperatur belassen. Der gebildete, harzartige Anteil wird abgetrennt und mit Chloroform über Kieselgel chromatographiert. Die Produkteluate werden im Vakuum zur Trockene eingeengt, und anschließend wird der Rohaustrag aus einem Aceton-Cyclohexan-Gemisch umkristallisiert. Man erhält NMR-spektroskopisches reines (3-Methyl-5-[2-(2-hydroxy-3-chlorpropoxy)-styryl]-isoxazol) vom Schmelzpunkt 67—68° C.

$^1$H-NMR-Spektrum ($CDCl_3$, TMS intern).
$\tau$ = 2,30 3,15 (m, 6H), 3,92 (s, 1H), 5,60—5,92 (m, 3H), 6,24 (d, J = 3,5 Hz, 2H), 6,77 (breites s, OH).

## Herstellung der erfindungsgemäßen Verbindungen

Die angegebenen Schmelzpunkte aller Beispiele beziehen sich stets auf die trans-isomeren Verbindungen.

## Beispiel 1

6,0 g 3-Methyl-5-[2-(2,3-epoxypropoxy)-styryl]-isoxazol und 5,0 g 2-(3-Trifluormethylphenyl)-1,1-dimethyläthylamin werden in 100 ml Isopropanol 10 Stunden auf Rückflußtemperatur erhitzt. Der nach dem Abdestillieren des Lösungsmittels verbleibende Rückstand wird über eine Kieselgel-Trockensäule (ca. 500 g Kieselgel/50 cm Säulenlauge) mit Chloroform chromatographiert. Der Eindampfrückstand der Produkte-Eluate wird in Äthanol/Äther gelöst und mit ätherischer Salzsäure bis zur vollständigen Salzfällung versetzt. Das ausgefallene 3-Methyl-5-[2-[2-hydroxy-3-(2-(3-trifluormethylphenyl)-1,1-dimethyläthylamino)-propoxy]-styryl]-isoxazol-hydrochlorid wird abgesaugt, mit trockenem Äther gewaschen und getrocknet. Ausbeute: 8,3 g (70% d. Th.) vom Schmp. 170—171° C.

$C_{26}H_{29}N_2O_3F_3 \cdot HCl$ (510,99)

| | | | | |
|---|---|---|---|---|
| Ber.: | C 61,11 | H 5,92 | N 5,48 | Cl 6,94 |
| Gef.: | C 60,8 | H 5,9 | N 5,7 | Cl 7,6 |

Die in der nachfolgenden Tabelle angegebenen Verbindungen werden in gleicher Weise aus 3-Methyl-5-[2-(2,3-epoxypropoxy)-styryl]-isoxazol und den entsprechenden Aminen erhalten. Sämtliche Verbindungen sind durch Elementaranalysen und $^1$H-NMR-Spektren charakterisiert.

Tabelle

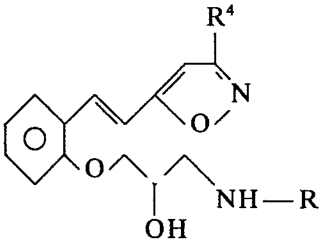

| Nr. | $R^4$ | R | Salzform | Schmelzp. (°C) |
|---|---|---|---|---|
| 2 | $-CH_2-CH_3$ | 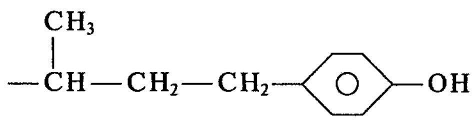 | freies Amin | 97–100 |
| 3 | $-CH_3$ | 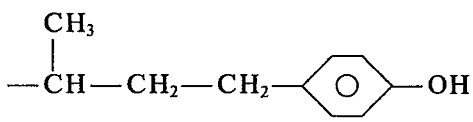 | freies Amin | 110–112 |
| 4 | $-CH_3$ | 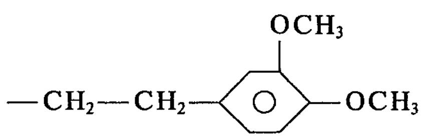 | freies Amin · 0,5 $H_2O$ | 80–83 |
| 5 | $-CH_3$ | 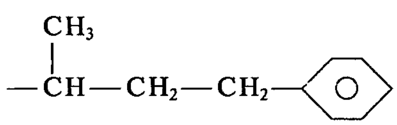 | freies Amin | 90–91 |
| 6 | $-CH_3$ | 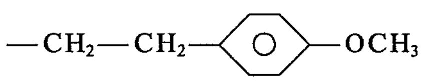 | HCl | 180–182 |
| 7 | $-CH_3$ | 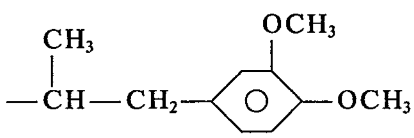 | freies Amin | 121–125 |
| 8 | $-CH_3$ | 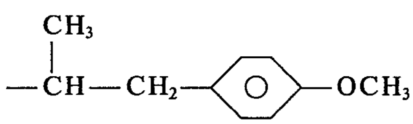 | 1/2 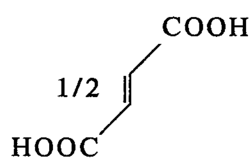 | 185–187 |
| 9 | $-CH_3$ | 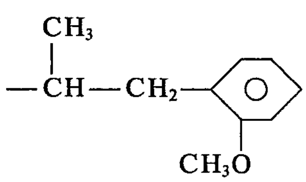 | 1/2 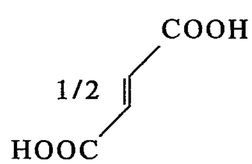 | 154–156 |
| 10 | $-CH_3$ | 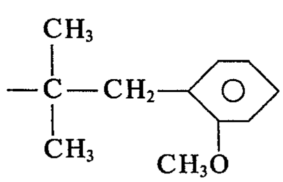 | HCl | 128–130 |

Fortsetzung

| Nr. | $R^4$ | R | Salzform | Schmelzp. (°C) |
|---|---|---|---|---|
| 11 | $-CH_3$ | 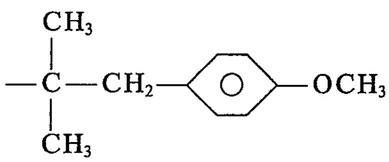 | HCl | 145–146 |
| 12 | $-CH_3$ | 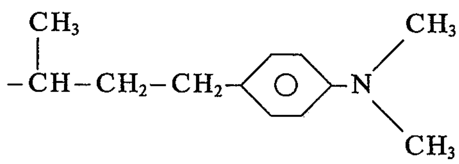 | freies Amin · 0,5 $H_2O$ | 103–105 |
| 13 | $-CH_3$ | 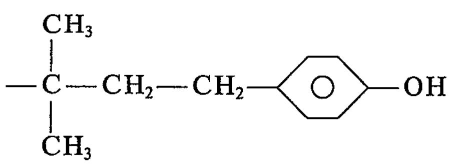 | freies Amin | 139–140 |
| 14 | $-CH_3$ | 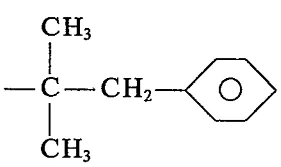 | HCl | 190 |
| 15 | $-CH_3$ | 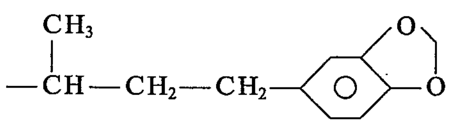 | HCl | 160–162 |
| 16 | $-CH_3$ | 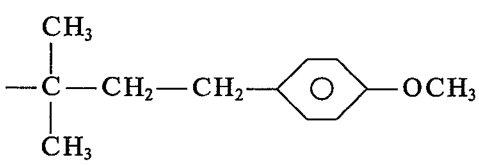 | freies Amin · 0,5 $H_2O$ | 83–85 |
| 17 | $-CH_3$ | 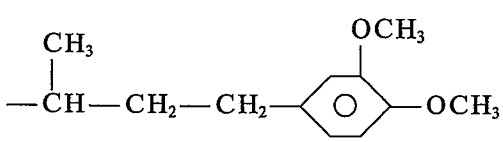 | freies Amin | 100–102 |
| 18 | $-CH_3$ | 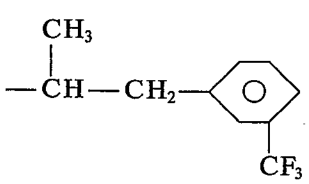 | freies Amin | 82–84 |
| 19 | $-CH_3$ | 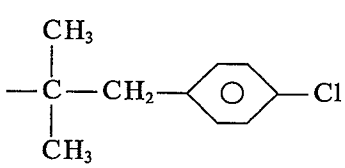 | HCl · 0,5 $H_2O$ | 179–181 |

Fortsetzung

| Nr. | $R^4$ | R | Salzform | Schmelzp. (°C) |
|---|---|---|---|---|
| 20 | $-CH_3$ | 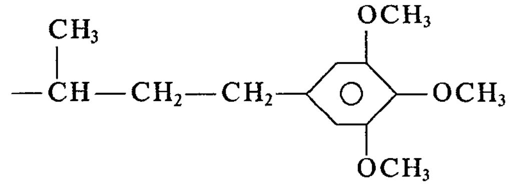 | 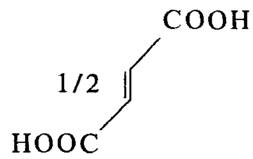 1/2 | 190–192 |
| 21 | $-CH_3$ | 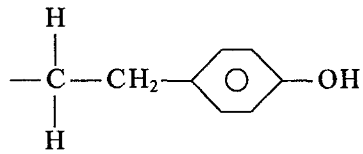 | 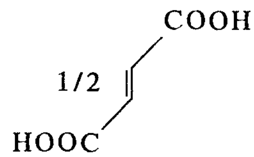 1/2 | 162 |

Formulierungsbeispiele, die in üblicher Weise hergestellt werden

1. Tabletten:

| | | |
|---|---|---|
| a) | Ein Wirkstoff der Formel I | 5 mg |
| | Lactose | 200 mg |
| | Methylcellulose | 15 mg |
| | Maisstärke | 50 mg |
| | Talkum | 11 mg |
| | Magnesiumstearat | 4 mg |
| | | 285 mg |
| b) | Ein Wirkstoff der Formel I | 20 mg |
| | Lactose | 178 mg |
| | Avicel | 80 mg |
| | Polywachs 6000 | 20 mg |
| | Magnesiumstearat | 2 mg |
| | | 300 mg |
| c) | Ein Wirkstoff der Formel I | 50 mg |
| | Polyvinylpyrrolidon (mittl. M. G. 25 000) | 170 mg |
| | Polyäthylenglykol (mittl. M. G. 4000) | 14 mg |
| | Hydroxypropylmethylcellulose | 40 mg |
| | Talkum | 4 mg |
| | Magnesiumstearat | 2 mg |
| | | 280 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M. G. 4000), Hydroxypropylmethylcellulose, Talkum und Magnesiumstearat vermischt und zu Tabletten à 280 mg verpreßt.

2. Beispiel für Dragees:

| | |
|---|---|
| Ein Wirkstoff der Formel I | 60 mg |
| Lactose | 90 mg |
| Maisstärke | 60 mg |
| Polyvinylpyrrolidon | 6 mg |
| Magnesiumstearat | 1 mg |
| | 217 mg |

Die Mischung der Wirkstoffsubstanz mit Lactose und Maisstärke wird mit einer 8%igen wäßrigen Lösung des Polyvinylpyrrolidons durch Sieb, 1,5 mm granuliert, bei 50°C getrocknet und nochmals durch Sieb, 1,0 mm, gerieben. Das so erhaltene Granulat wird mit Magnesiumstearat gemischt und zu

Drageekernen verpreßt. Die erhaltenen Drageekerne werden in üblicher Weise mit einer Hülle überzogen, die im wesentlichen aus Zucker und Talkum besteht.

3. Kapselformulierung:

| | |
|---|---|
| Ein Wirkstoff der Formel I | 5,0 mg |
| Magnesiumstearat | 2,0 mg |
| Milchzucker | 19,3 mg |

4. Injektionslösung:

| | |
|---|---|
| Ein Wirkstoff der Formel I | 10 mg |
| Natriumchlorid | 9 mg |
| destilliertes Wasser, q. s. auf 1,0 ml | |

**Patentansprüche**

1. Verbindungen der allgemeinen Formel (I)

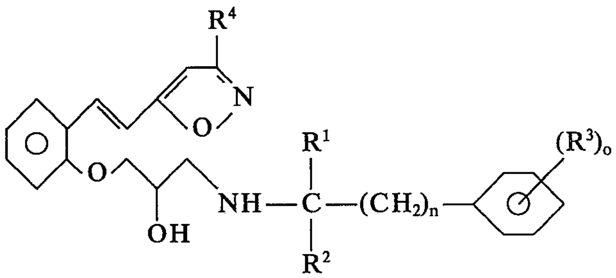

(I)

in der n die Zahlen 1 oder 2, o die Zahlen 1 bis 3, $R^1$ und $R^2$ ein Wasserstoffatom oder eine geradkettige oder verzweigte Alkylgruppe mit 1 bis 5 C-Atomen, $R^3$ ein Wasserstoffatom, eine Hydroxygruppe, ein Halogenatom, eine Alkyl-, Alkoxy- oder Alkylthiogruppe mit 1 bis 5 C-Atomen, gegebenenfalls jeweils in der Alkylgruppe 1- bis 3fach durch Halogenatome oder einfach durch eine Hydroxy- oder Alkoxygruppe mit 1 bis 3 C-Atomen substituiert, eine Alkenyl-, Alkinyl-, Alkinyloxy- oder Cycloalkoxygruppe mit jeweils 2 bis 6 C-Atomen und 3 bis 8 C-Atomen im Ring, eine Aminogruppe, gegebenenfalls ein- oder zweifach mit einer Alkylgruppe mit 1 bis 5 C-Atomen substituiert, bedeuten, wobei entsprechend der Bedeutung von o mehrere Reste $R^3$ gleich oder verschieden sein können, oder $R^3$ bedeutet eine Methylendioxygruppe oder eine Alkylengruppe mit 3 oder 4 Kohlenstoffatomen, und $R^4$ einen Alkylrest mit 1 bis 4 C-Atomen bedeutet und ihre Säureadditionssalze.

2. 3-Methyl-5-[2-[2-hydroxy-3-(3-(4-hydroxyphenyl)-1-methylpropylamino)-propoxy]-styryl]-isoxazol.

3. 3-Methyl-5-[2-[2-hydroxy-3-(2-(3,4-dimethoxyphenyl)-1-äthylamino)-propoxy]-styryl]-isoxazol.

4. Verfahren zur Herstellung von Verbindungen der Formel (I) nach Anspruch 1, dadurch gekennzeichnet, daß man ein 3-Alkyl-5-styryl-isoxazol der allgemeinen Formel (II)

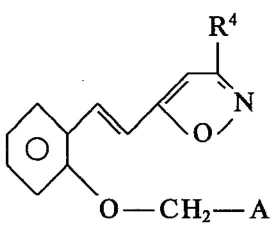

(II)

in der $R^4$ die für Formel I angegebenen Bedeutungen hat und A den Rest

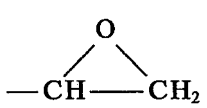

oder

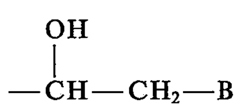

wobei B für eine nukleofuge Abgangsgruppe steht, bedeutet, mit einem Amin der allgemeinen Formel (III)

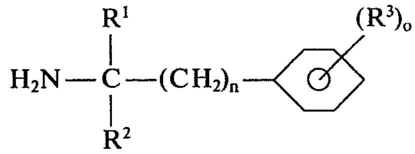

(III)

in der $R^1$, $R^2$, $R^3$, n und o die für Formel I angegebenen Bedeutungen haben, zweckmäßigerweise in einem Lösungsmittel und gegebenenfalls in Gegenwart eines säurebindenden Mittels in an sich bekannter Weise umsetzt und die erhaltene Verbindung gegebenenfalls in das Säureadditionssalz einer physiologisch verträglichen Säure überführt.

5. Therapeutische Mittel, gekennzeichnet durch einen Gehalt an einer Verbindung der Formel I oder ihres physiologisch verträglichen Säureadditionssalzes als Wirkstoff neben üblichen Trägerstoffen und Verdünnungsmitteln.

6. Verbindung nach Anspruch 1 zur Verwendung als Arzneimittel bei der Behandlung der Hypertonie, der coronaren Herzkrankheit und von Herzrhythmusstörungen.

## Claims

1. A compound of the general formula (I)

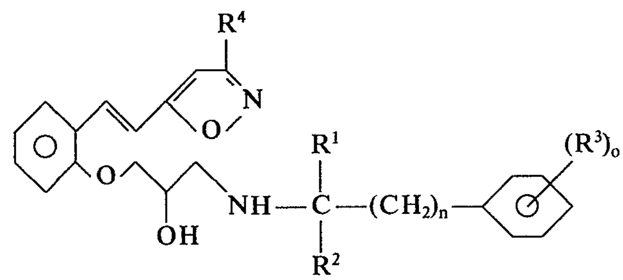

(I)

where n is 1 or 2, o is 1, 2 or 3, $R^1$ and $R^2$ are each hydrogen or straight-chain or branched alkyl of 1 to 5 carbon atoms, $R^3$ is hydrogen, hydroxyl, halogen, alkyl, alkoxy or alkylthio of 1 to 5 carbon atoms (the lastmentioned three groups each being unsubstituted, or mono-, di- or tri-substituted by halogen or mono-substituted by hydroxyl or alkoxy of 1 to 3 carbon atoms), alkenyl, alkynyl, alkynyloxy or cycloalkoxy, each of 2 to 6 carbon atoms in the alkyl and of 3 to 8 carbon atoms in the ring, or amino which is unsubstituted or is mono- or di-substituted by alkyl of 1 to 5 carbon atoms, and depending on the meaning of o, the $R^3$'s may be identical or different, or $R^3$ is methylendioxy or alkylene of 3 or 4 carbon atoms, and $R^4$ is alkyl of 1 to 4 carbon atoms, and its addition salts wiht acids.

2. 3-Methyl-5-[2-[2-hydroxy-3-(3-(4-hydroxyphenyl)-1-methylpropylamino)-propoxy]-styryl]-isoxazole.

3. 3-Methyl-5-[2-[2-hydroxy-3-(2-(3,4-dimethoxyphenyl)-1-ethylamino)-propoxy]-styryl]-isoxazole.

4. A process for the preparation of a compound of the formula (I) as claimed in claim 1, wherein a 3-alkyl-5-styryl-isoxazole of the general formula (II)

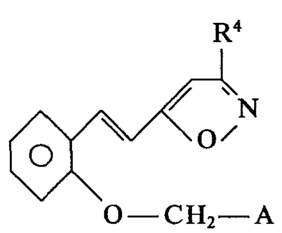

(II)

where $R^4$ has the meanings given for formula I and A is

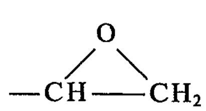

or

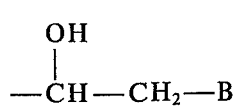

B being a nucleofugic leaving group, is reacted with an amine of the general formula (III)

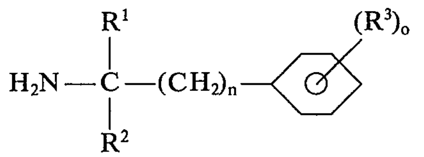

(III)

where $R^1$, $R^2$, $R^3$, n and o have the meanings given for formula I, in a conventional manner, advantageously in a solvent and in the presence or absence of an acid acceptor, and the compound obtained is converted, if required, into an addition salt with a physiologically tolerated acid.

5. A therapeutic agent which contains a compound of the formula I, or a physiologically tolerated addition salt thereof with an acid, as the active compound, together with conventional carriers and diluents.

6. A compound as claimed in claim 1 for use as a pharmaceutical in the treatment of hypertonia, coronary heart disease and cardiac arrhythmias.

**Revendications**

1. Composés de formule générale (I)

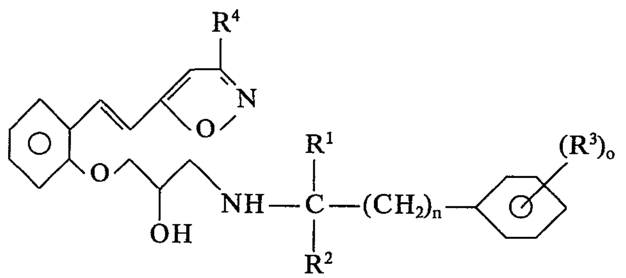

(I)

dans laquelle n représente le nombre 1 ou 2, o un nombre de 1 à 3, $R^1$ et $R^2$ un atome d'hydrogène ou un groupe alkyle ayant 1 à 5 atomes C, à chaine droite ou ramifiée, $R^3$ un atome d'hydrogène, un groupe hydroxy, un atome d'halogène, un groupe alkyle, alcoxy ou alkylthio à 1 à 5 atomes C, éventuellement chacun substitué dans le groupe alkyle, 1 à 3 fois, par un atome d'halogène ou une fois par un groupe hydroxy ou alcoxy à 1 à 3 atomes C, un groupe alcényle, alkinyle, alkinyloxy ou cycloalcoxy ayant chacun 2 à 6 atomes C et 3 à 8 atomes C dans le noyau, un groupe amino, éventuellement substitué une ou deux fois par un groupe alkyle à 1 à 5 atomes C, plusieurs restes $R^3$ pouvant être identiques ou différents selon la signification de o, ou $R^3$ représente un groupe méthylènedioxyde ou un groupe alkylène à 3 ou 4 atomes de carbone, et $R^4$ représente un reste alkyle à 1 à 4 atomes C, et leurs sels d'addition d'acide.

2. 3-méthyl-5-[2-[2-hydroxy-3-(3-(4-hydroxyphényl)-1-méthylpropylamino)-propoxy]-styryl]-isoxazole.

3. 3-méthyl-5-[2-[2-hydroxy-3-(2-(3,4-diméthoxyphényl)-1-éthylamino)-propoxy]-styryl]-isoxazole.

4. Procédé de préparation de composés de formule (I) selon la revendication 1, caractérisé par le fait qu'on fait réagir, de manière connue en soi, de préférence dans un solvant et éventuellement en présence d'un agent liant les acides, un 3-alkyl-5-styryl-isoxazole de formule générale (II)

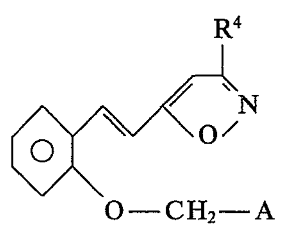

(II)

dans laquelle $R^4$ a les significations indiquées pour la formule I et A représente le reste

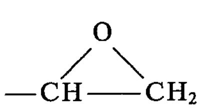

ou

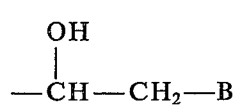

B représentant un groupe de départ nucléofuge, avec une amine de formule générale III

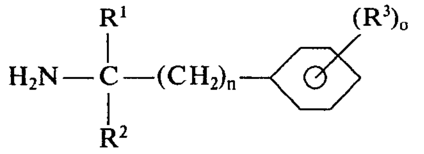

(III)

dans laquelle $R^1$, $R^2$, $R^3$, n et o ont les significations données par la formule I et on convertit éventuellement le composé obtenu en le sel d'addition d'acide d'un acide physiologiquement compatible.

5. Agent thérapeutique caractérisé par le fait qu'il contient comme principe actif, outre des véhicules et diluants usuels, un composé de formule I ou son sel d'addition d'acide physiologiquement compatible.

6. Composé selon la revendication 1 pour l'utilisation comme médicament pour le traitement de l'hypertonie, des maladies coronariennes et des troubles du rythme cardiaque.